# NEUE EUROPÄISCHE PATENTSCHRIFT

(11) **EP 1 465 687 B2**
(45) Veröffentlichungstag und Bekanntmachung des Hinweises auf die Entscheidung über den Einspruch: **23.05.2012**
(45) Hinweis auf die Patenterteilung: 04.04.2007
(21) Anmeldenummer: 02702217.7
(22) Anmeldetag: 15.01.2002
(51) Int. Cl.: A61M 1/16, A61K 9/10

(54) **KARTUSCHE ZUR HERSTELLUNG EINES ACETATDIALYSEKONZENTRATS AUS EINER HOCHVERDICHTETEN KONZENTRATVORSTUFE UND VERFAHREN ZUR AUFBEREITUNG DER KARTUSCHE**
CARTRIDGE FOR PREPARATION OF AN ACETATE DIALYSIS CONCENTRATE FROM A HIGHLY DENSIFIED CONCENTRATE AND METHOD OF PROCESSING THE CARTRIDGE
CARTOUCHE POUR PRODUIRE UN CONCENTRE POUR DIALYSE D'ACETATE A PARTIR D'UN PRECURSEUR DE CONCENTRE FORTEMENT DENSIFIE ET PROCEDURE POUR LE TRAITEMENT DE CARTOUCHE

(43) Veröffentlichungstag der Anmeldung: 13.10.2004
(73) Patentinhaber: Sterisafe GmbH, 14547 Buchholz (DE)
(72) Erfinder: EIGENDORF, Hans-Günther, 15526 Bad Saarow-Pieskow (DE); PIPPERT, Manfred, 61479 Glashütten (DE); HILDMANN, Uwe, 98708 Gehren (DE)
(74) Vertreter: Flosdorff, Jürgen
(86) Internationale Anmeldenummer: PCT/DE2002/000095
(87) Internationale Veröffentlichungsnummer: WO 2003/059416

(56) Entgegenhaltungen:
- EP-A- 0 456 928
- EP-A- 0 575 970
- EP-A- 0 697 220
- EP-A1- 0 605 395
- WO-A-01/02036
- DE-A- 19 931 077
- DE-C- 4 211 455
- US-A- 4 137 168
- US-A- 4 336 881
- US-A- 6 149 294

## Beschreibung

Die Erfindung betrifft eine neuartige Bereitstellungsform der notwendigen Bestandteile von Dialysierflüssigkeiten für den Einsatz in der Hämodialyse, wobei die Bestandteile nicht entsprechend Stand der Technik als Flüssigkonzentrat oder als Trockenkonzentrat an die Dialyseeinrichtung gebracht werden, sondern in Form einer Aufschlämmung mit höherer Stoffdichte. Der Rohstoffanteil in der Aufschlämmung liegt je nach Spezifikation zwischen 72% und 90 % der Gesamtmasse. Aus den mit der Aufschlämmung gefüllten Behältern, z.B. Kartuschen, werden mit Hilfe einer in dieser Erfindung beschriebenen Vorrichtung am Dialyseplatz Dialysekonzentrate hergestellt, die in üblicher Weise von Dialysemaschinen zu Dialysierflüssigkeit gemischt werden können.

Aus Dialysekonzentraten wird vor Durchführung einer Dialyse an einem Patienten eine Dialysierflüssigkeit hergestellt, indem das oder die Dialysekonzentrat(e) mit einer vorbestimmten Menge Wasser geeigneter Qualität gemischt werden.

Dialysierflüssigkeit kann im Allgemeinen folgende Bestandteile haben, deren An- oder Abwesenheit und quantitativen Verhältnisse je nach Anwendungsfall unterschiedlich sein können: Natriumchlorid, Natriumhydrogencarbonat, Glucose, Kaliumchlorid, Calciumchlorid, Magnesiumchlorid, Essigsäure, Salzsäure, Natriumacetat, Calciumgluconat, Ascorbinsäure, Zitronensäure, Hydrate dieser Verbindungen, Wasser geeigneter Qualität für die Dialyse und gegebenenfalls weitere Inhaltsstoffe.

Bei der allgemein als Bicarbonat-Hämodialyse bezeichneten Anwendungsform werden ein sogenanntes "Saures Konzentrat" und ein sogenanntes "Basisches Konzentrat" in definierten Anteilen mit einer vorbestimmten Menge Wasser zur Dialysierflüssigkeit gemischt. Das Basische Konzentrat enthält immer Natriumhydrogencarbonat oder eine in Hydrogencarbonat umzuwandelnde Komponente, gegebenenfalls auch weitere Bestandteile ausschließlich der Erdalkalisalze und Säuren.

Das Saure Konzentrat kann alle notwendigen Bestandteile außer Natriumhydrogencarbonat enthalten.

In der sogenannten Acetatdialyse wird ein Acetatkonzentrat verwendet, das allein durch Mischen mit Wasser in definierten Verhältnissen eine Dialysierflüssigkeit ergibt.

Die Inhaltsstoffe der Dialysekonzentrate werden üblicherweise in einer Produktionsanlage in definierten Mengen Wasser zu einer echten Lösung gelöst und als Dialysekonzentrat zu einer Dialysestation transportiert. Wegen des hohen Wasseranteils in den Lösungen ist dies mit einem beträchtlichen Transport- und Lageraufwand verbunden

Deshalb wird seit einiger Zeit die bevorzugte basische Komponente zur Herstellung der Dialysierflüssigkeit - das Natriumhydrogencarbonat - zunehmend als Pulver an das Dialysegerät gebracht und dort unter Hinzufügen von Wasser zum Basischen Konzentrat gelöst, wobei sowohl Batch- als auch Onlineverfahren zum Einsatz kommen.

Es ist auch bereits in geringem Umfang Praxis, die Bestandteile des Sauren Konzentrates zur Herstellung der Dialysierflüssigkeit in Pulverform am Ort der Dialyse zur Verfügung zu stellen, wodurch der Transport- und Lageraufwand verringert ist. Die einzelnen Bestandteile müssen dann vor Ort aber mit hoher Genauigkeit dosiert werden. Dies erfordert einen erheblichen technischen und personellen Aufwand. Es ist unter den gegebenen Bedingungen einer Dialyseeinrichtung nicht sicher zu verifizieren, ob die einzelnen Bestandteile tatsächlich in dem vorgegebenen Mischungsverhältnis enthalten sind. Außerdem ist dieses Verfahren aus hygienischen Gründen umstritten. Hieraus ergeben sich hohe Risiken für den Patienten, da aus fehlerhafter Zusammensetzung der Dialysierflüssigkeit gesundheitliche Schäden, im Extremfall mit Todesfolge resultieren können.

DE-A- 199 31 077 und WO 01/02036 offenbaren eine Kartusche mit einem Sauren Dialysekonzentrat, bestehend aus einer wäßrigen Aufschlämmung ungelöster Stoffe und einer Lösung der restlichen, für eine Bicarbonatdialyse notwendigen Bestandteile. Außerdem beschreiben diese Druckschriften eine Vorrichtung zur Aufbereitung der Kartuschen mit einem Konzentratbehälter, einem Batchgefäß, verbindenden Leitungen, einem Wasserzulauf und einem Ablauf sowie einer Pumpe zur Zirkulation des zugeführten Wassers. In den Druckschriften fehlt jeglicher Hinweis auf zu wählende Rohstoffanteile und Wasseranteile.

EP-A-0 456 928 offenbart eine pastöse Dialysekontratzusammensetzung, die physikalisch instabil ist und vor Entnahme von Teilen zum Zwecke der Homogenisierung umgerührt werden muß.

Die vorliegende Erfindung ist eine Grundlage für die kostengünstige industrielle Fertigung einer neuartigen Vorstufe von Acetatkonzentraten und deren Verwendung am Dialyseplatz zur Herstellung von Dialysekonzentrat. Das gebildete Dialysekonzentrat kann nachfolgend von der Dialysemaschine in üblicher Weise zur Herstellung von Dialysierflüssigkeit verwendet werden.

Die genannte Vorstufe eines Dialysekonzentrates hat eine höhere Stoffdichte, als jede andere entsprechend Stand der Technik bekannte Bereitstellungsform der Bestandteile für die Hämodialyse, woraus Einsparungseffekte bei Transport, Lagerung und Handling resultieren, ohne die Sicherheit der Dialysebehandlung einzuschränken.

Diese Aufgaben werden erfindungsgemäß durch die Merkmale des Patentanspruchs 1 gelöst. Vorteilhafte Weiterbildungen dieses Aspekts der Erfindung sind in den abhängigen Ansprüchen 2 bis 16 gekennzeichnet.

Die im Patentanspruch 1 gekennzeichnete Erfindung sieht vor, dass alle Inhaltstoffe des Azetat Konzentrats in einem Behältnis als Aufschlämmung vorliegen. Diese bevorzugte Variante minimiert das Volumen der zu transportierenden, zu lagernden und zu handhabenden Bestandteile des Konzentrats gegenüber herkömmlichen Dialysekonzentraten, wobei die physiologisch notwendige Homogenität des an der Dialysemaschine hergestellten Konzentrats durch die kontrollierte industrielle Fertigung einerseits und die im Anspruch 4 beschriebene Vorrichtung andererseits leicht gewährleistet werden kann.

### Beispiel einer Sauren Kartusche

Es wird eine Lösung folgender Zusammensetzung hergestellt:
- 35,58 g: Magnesiumchlorid-Hexahydrat PH.EUR.
- 104,4 g: Kaliumchlorid PH.EUR.
- 90,04 g: Calciumchlorid-Dihydrat PH.EUR.
- 73,62 g: Essigsäure 100 % PH.EUR.
- 385,0 g: Glucose-Monohydrat PH.EUR.
werden in einem 1 Liter-Meßkolben mit aqua purificata unter Rühren zu 1 Liter gelöst.

600 ml dieser Lösung und 1264 g Natriumchlorid PH.EUR. werden in ein flaschenähnliches Kunststoffgefäß dosiert. Es entsteht ein ausgeprägtes Zweiphasensystem, bei dem der flüssigkeitsdurchtränkte Bodensatz vorwiegend Natriumchlorid und die klare überstehende Flüssigkeit vorwiegend alle anderen Bestandteile eines Sauren Dialysekonzentrares enthält. Das Gesamtvolumen dieser Aufschlämmung beträgt 1200 bis 1210 ml. Der Rohstoffanteil in diesem Beispiel beträgt 83,11 %, der Wasseranteil entsprechend 16,89 % .

Das Konzentrat kann von einer Dialysemaschine in bekannter Weise zu 210 l Dialysierflüssigkeit der nachfolgenden Zusammensetzung gemischt werden:

| | |
|---|---|
| Natrium | 138,00 mmol/l |
| Kalium | 4,00 mmol/l |
| Calcium | 1,75 mmol/l |
| Magnesium | 0,50 mmol/l |
| Acetat | 3,50 mmol/l |
| Chlorid | 111,50 mmol/l |
| Bicarbonat | 31,50 mmol/l |
| Glucose | 5,55 mmol/l (entspr. 1 g/l) |

Die in Patentanspruch 1 gekennzeichnete Erfindung sieht das Prinzip der sauren Kartusche für eine Acetat-Kartusche vor.

Die Verwendung der vorstehend bezeichneten Kartuschen ermöglicht es, die aus der herkömmlichen industriellen Flüssigkonzentratfertigung bekannten Vorteile hinsichtlich Dosiergenauigkeit, Prüfbarkeit bis zur gesetzlich geforderten Rückverfolgbarkeit einzuhalten und dennoch eine wesentliche Volumen- und Massereduktion zu erreichen.

Die Erfindung erlaubt, die im Dialysekonzentrat enthaltenen Komponenten mit geringem Masseanteil bei der industriellen Fertigung kostengünstig als Lösung aus einem geprüften grösseren Batch mit notwendiger und nachweisbarer Genauigkeit in den Kartuschenkörper zu dosieren. Dadurch ist gewährleistet, dass das aus der Kartusche hergestellte Dialysekonzentrat und die nachfolgend von der Dialysemaschine gemischte Dialysierflüssigkeit eine gleichbleibende, kontrollierte Qualität und Patientensicherheit aufweist.

Die Aufschlämmung enthält vorzugsweise Natriumchlorid als festen Bestandteil, wobei es zweckmäßig sein kann, dass auch noch weitere Bestandteile in fester Form vorliegen. Die festen Bestandteile sind in einer Lösung der übrigen Bestandteile aufgeschlämmt. Aus dieser Aufschlämmung wird in der nachfolgend noch beschriebenen Vorrichtung an oder in der Dialysemaschine durch Zugabe einer definierten Menge Wasser geeigneter Qualität im Batch-Verfahren ein flüssiges Konzentrat - vergleichbar den üblichen Fertigkonzentraten - hergestellt, dass mit den bestehenden Dosier- und Sicherheitssystemen marktüblicher Dialysegeräte zu Dialysierflüssigkeit verarbeitet werden kann.

Das zur Herstellung der Acetatkartusche verwendete Behältnis kann ein aus Kunststoff oder Glas bestehender formstabiler Körper oder ein flexibler Beutel sein , der nach der Befüllung mit der Aufschlämmung flüssigkeitsdicht und gasdicht verschlossen wird. Dabei ist erfindungsgemäß vorgesehen, dass Zufluss und Abfluss der Kartuschen so gestaltet sind, dass in der strömenden Flüssigkeit befindliche ungelöste Stoffbestandteile beim Austritt aus der Kartusche durch ein Filter zurückgehalten werden, oder die Vorrichtung gemäß Anspruch 4 eine solche Filterfunktion aufweist.

Die Erfindung betrifft ferner diese Vorrichtung zum Aufbereiten der Kartuschen zu Konzentrat. Die Vorrichtung enthält eine Aufnahmeeinrichtung für die Kartusche, die ein flüssigkeitsdichtes Einfügen in die Zirkulation ermöglicht. Weiterhin gehören ein Batch-Gefäss, dass vorzugsweise mit einem Füllstandsensor versehen ist, der nach Erreichen eines vorbestimmten Füllstands den weiteren Wasserzulauf unterbricht, Leitungen, die Einlassöffnung und Auslauföffnung der Kartusche mit einer Einlassöffnung und einer Auslassöffnung des Batch-Gefäßes verbinden, einen Wasserzulauf und einen Dialysekonzentratablauf, sowie eine Pumpe, die das zugeführte Wasser bzw. die Lösung vorzugsweise entgegen der Schwerkraft solange durch das Batch-system zirkulieren lässt, bis sich alle festen Bestandteile der Aufschlämmung aufgelöst haben und eine homogene Lösung im System vorliegt. Dies kann von einer Steuereinheit mittels Sensoren überwacht oder zeitgesteuert werden und in einem Signal zur Öffnung des Auslassventils münden.

Diese Vorrichtung kann in ein Dialysegerät integriert sein oder als ein Zusatzgerät an dem Dialysegerät angeordnet werden oder unabhängig vom Dialyseplatz zur Konzentratherstellung verwendet werden.

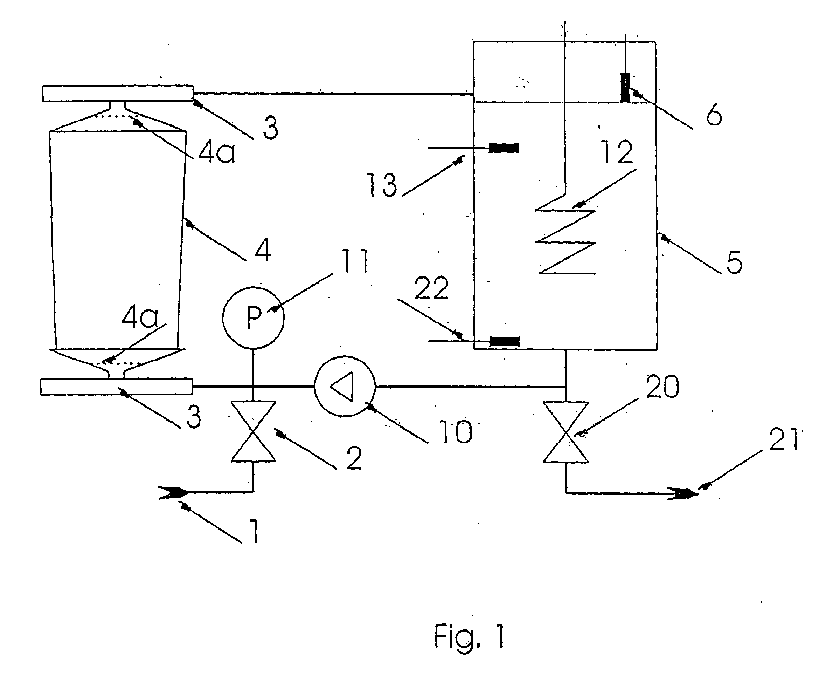

Weitere Einzelheiten der Vorrichtung zum Aufbereiten einer Kartusche ergeben sich aus der nachfolgenden Beschreibung einer bevorzugten Ausführungsform sowie anhand der beigefügten Zeichnung. Diese zeigt auf rein schematische Weise eine derartige Vorrichtung.

Über einen Anschluss 1 wird Wasser geeigneter Qualität in die Vorrichtung eingelassen. Bevorzugt handelt es sich um Wasser aus dem Dialysegerät, das entgast und temperiert ist. Wenn derartiges Wasser nicht zur Verfügung steht, kann anderweitig verfügbares Wasser geeigneter Qualität verwendet werden, dass in der Vorrichtung vorzugsweise erwärmt werden kann.

Nach Anschluss einer Kartusche 4 an die beiden Anschlüsse 3 wird ein Zulaufventil 2 geöffnet, so dass Wasser vorzugsweise von unten in die Kartusche eintritt. Die beiden Anschlüsse 3 sind vorzugsweise an gegenüberliegenden Seiten der Kartusche angebracht, können aber auch nebeneinander auf der gleichen Seite oder beliebig anders lokalisiert sein. An Zu- und Ablauf der Kartusche sind jeweils Filter 4a vorgesehen, die das Austreten ungelöster Bestandteile aus der Kartusche verhindern. Alternativ kann die Filterfunktion in die Batchvorrichtung verlagert sein. Nach Einströmen von Wasser in die Kartusche fließt die entstehende Lösung in ein Batch-Gefäss 5. Ein Füllstandsensor 6 beendet den Wasserzulauf. Der Füllstandsensor ermöglicht die Festlegung auf unterschiedliche, genau definierte Verdünnungsverhältnisse, wobei das gesamte Batch-Volumen der Summe der Volumina von Batch-Gefäss 5, Kartusche 4 und dem zugehörigen Schlauchsystem entspricht. Ersatzweise wird bei einer anderen Version das Volumen der entleerten Kartusche dem Batchvolumen nicht zugerechnet.

Der Füllstandsensor 6 ist vorzugsweise als Leitfähigkeitssensor ausgebildet und ermöglicht somit eine zusätzliche Kontrolle der Lösungskonzentration.

Nach Füllung des gesamten Batch-Volumens wird eine Pumpe 10 aktiviert, die die Flüssigkeit in dem System zirkulieren lässt, damit die vollständige Auflösung der Feststoffe beschleunigt und zeitabhängig Homogenität bewirkt.

Als Sicherheitsüberwachung ist ein Drucksensor 11 vorgesehen. Die Vorrichtung enthält ferner bevorzugt eine Heizung 12, um das Lösen der Feststoffe zu beschleunigen, und einen Temperatursensor 13. Der Temperatursensor 13 dient einerseits der Heizungsregeiung und kann andererseits in Verbindung mit der Leitfähigkeitsmesszelle 6 mit der notwendigen Genauigkeit die Vollständigkeit der Auflösung der Feststoffe nachweisen.

Nach dem vollständigen Lösen der Feststoffe und homogener Verteilung der Bestandteile ist ein verwendungsfähiges Dialysekonzentrat entstanden. Es wird nun ein Auslassventil 20 geöffnet, um das Dialysekonzentrat durch die Auslassleitung 21 zum Dialysegerät weiterzuleiten.

Das Auslassventil 20 kann vor oder nach der Pumpe 10 angeordnet sein.

Außerdem kann die Vorrichtung einen Niveausensor 22 enthalten, der einen minimalen Flüssigkeitsstand meldet.

## Patentansprüche

1. Behältnis mit Bestandteilen eines Acetatdialysekonzentrates, wobei die zur Herstellung von Dialysierflüssigkeit für die Acetatdialyse essentiellen Bestandteile in diesem als "Acetatkartusche" bezeichneten Behältnis vorliegen und dabei partiell in Wasser gelöst sind, während erhebliche Anteile ungelöst bleiben und mit der Lösung eine Aufschlämmung bilden, wobei eine Aufschlämmung wenigstens eines pulver- oder granulatförmigen Bestandteils und der in Wasser gelösten übrigen Bestandteile des Acetatkonzentrats vorliegt,
**dadurch gekennzeichnet,**
**dass** die Bestandteile mit einem Rohstoffanteil von 72% bis 90% der Gesamtmasse vorliegen und
**dass** der Wasseranteil in Abhängigkeit von der gewünschten Zusammensetzung von der gewünschten Zusammensetzung des Konzentrates 28% bis 10% der Gesamtmasse beträgt.

2. Behältnis nach Anspruch 1,
**dadurch gekennzeichnet,**
**dass** eine Aufschlämmung mehrerer pulver- oder granulatförmiger Bestandteile und der in Wasser gelösten übrigen Bestandteile des Acetatkonzentrates vorliegt.

3. Behältnis nach den Ansprüchen 1 und 2,
**dadurch gekennzeichnet,**
**dass** das Behältnis eine Kartusche ist, die in eine Vorrichtung zur Herstellung von Dialysekonzentrat nach Anspruch 4 einsetzbar ist.

4. Vorrichtung zur Aufbereitung von Kartuschen nach Anspruch 1,
**gekennzeichnet durch**
eine lösbar eingesetzte Kartusche (4) mit den als Aufschlämmung vorliegenden Bestandteilen eines Dialysekonzentrats nach den Ansprüchen 1 bis 2, ein Batch-Gefäss (5), verbindende Leitungen, einen Wasserzulauf (1) und einen Ablauf (21) sowie eine Pumpe (10) zur Zirkulation des zugeführten Wassers bzw. des sich bildenden Konzentrats, um die festen Bestandteile vollständig aufzulösen und homogen im Zirkulationssystem zu verteilen.

5. Vorrichtung nach Anspruch 4,
**dadurch gekennzeichnet,**
**dass** die Vorrichtung in ein Dialysegerät integriert ist.

6. Vorrichtung nach Anspruch 4,
**dadurch gekennzeichnet,**
**dass** die Vorrichtung als Zusatzgerät mit einem Dialysegerät verbindbar ist.

## Claims

1. A container with components of an acetate dialysis concentrate, wherein the essential components for producing dialysis liquid for acetate dialysis are present in this container, referred to as an "acetate cartridge" and are partially dissolved in water, whilst considerable proportions remain undissolved and form a suspension with the solution, wherein there is a suspension of at least one pulverulent or granular component and the remaining components of the acetate concentrate dissolved in water,
**characterised in**
**that** the components are present with a raw material proportion of 72% to 90% of the total mass and that the proportion of water is 28% to 10% of the total mass, depending on the desired composition of the concentrate.

2. A container as claimed in Claim 1,
**characterised in**
**that** there is a suspension of a plurality of pulverulent or granular components and the other components of the acetate concentrate dissolved in water.

3. A container as claimed in Claims 1 and 2,
**characterised in**
**that** the container is a cartridge, which may be inserted into an apparatus for producing dialysis concentrate as claimed in Claim 4.

4. Apparatus for preparing cartridges as claimed in Claim 1
**characterised by**
a releasably inserted cartridge (4) with the components, present in the form of a suspension, of a dialysis concentrate as claimed in Claims 1 to 2, a batch vessel (5), connecting conduits, a water supply (1) and a discharge (21) and a pump (10) for circulating the supplied water or the concentrate which forms in order to completely dissolve the solid components and to distribute them homogeneously in the circulation system.

5. Apparatus as claimed in Claim 4,
**characterised in that** the apparatus is integrated into a dialysis device.

6. Apparatus as claimed in Claim 4,
**characterised in**
**that** the apparatus is connectable to a dialysis device as an auxiliary device.

## Revendications

1. Récipient avec des composants d'un concentré de dialyse à l'acétate, dans lequel les composants essentiels pour la préparation de liquide de dialyse pour la dialyse à l'acétate sont présents dans ce récipient désigné comme « cartouche d'acétate » et sont ce faisant partiellement dissous dans l'eau, tandis que des parts considérables restent non dissoutes et forment une suspension avec la solution, en ce qu'on a une suspension d'au moins un composant pulvérulent ou granuleux et des autres composants dissous dans l'eau du concentré d'acétate,
**caractérisé en ce que**
les composants sont présents avec une proportion brute de matière de 72 % à 90 % de la masse totale et
**en ce que** la proportion d'eau est de 28 % à 10 % de la masse totale en fonction de la composition désirée du concentré.

2. Récipient selon la revendication 1,
**caractérisé en ce qu'**on a une suspension de plusieurs composants pulvérulents ou granuleux et des autres composants dissous dans l'eau du concentré d'acétate.

3. Récipient selon les revendications 1 et 2,
**caractérisé en ce que** le récipient est une cartouche qui est utilisable dans un dispositif pour la préparation de concentrés pour dialyse selon la revendication 4.

4. Dispositif pour l'élaboration des cartouches selon la revendication 1,
**caractérisé par** une cartouche (4) mise en place de façon démontable avec les composantes, présents comme suspension, d'un concentré pour dialyse selon les revendications 1 à 2, une cuve pour charges (5), des conduites de raccordement, une arrivée d'eau (1) et une sortie (21) ainsi qu'une pompe (10) pour la circulation de l'eau acheminée, respectivement du concentré qui se forme, pour dissoudre complètement les composants solides et les répartir de façon homogène dans le système de circulation.

5. Dispositif selon la revendication 4,
**caractérisé en ce que** le dispositif est intégré dans un appareil de dialyse.

6. Dispositif selon la revendication 4,
**caractérisé en ce que** le dispositif peut être raccordé à un appareil de dialyse comme appareil supplémentaire.
